# EUROPEAN PATENT SPECIFICATION

(11) **EP 1 977 684 B1**
(45) Date of publication and mention of the grant of the patent: **06.07.2016**
(21) Application number: 08001917.7
(22) Date of filing: 01.02.2008
(51) Int. Cl.: A61B 1/00, A61B 1/012, A61B 1/018, A61B 1/31

(54) **Endoscope**
Endoskop
Endoscope

(30) Priority: 16.03.2007 JP 2007069200
(43) Date of publication of application: 08.10.2008
(73) Proprietor: FUJIFILM Corporation, Tokyo (JP)
(72) Inventor: Koga, Takehiko, Saitama-shi Saitama (JP)
(74) Representative: Klunker . Schmitt-Nilson . Hirsch

(56) References cited:
- EP-A- 1 654 977
- EP-A- 1 695 657
- EP-A1- 1 857 040
- WO-A-2006/129419
- DE-A1- 3 326 648
- DE-A1-102004 023 457

## Description

### BACKGROUND OF THE INVENTION

### 1. Technical Field

The invention relates to an endoscope according to the preamble of claim 1.

### 2. Description of the Related Art

In an endoscope, a balloon is attached for various purposes. For example, in an endoscope for observing a deep alimentary canal such as a small intestine or a large intestine, the balloon is attached to the outer circumferential surface of the front end of the insertion portion and the balloon is inflated to fix the insertion portion to the alimentary canal. Additionally, in an ultrasonic endoscope, the balloon is attached so as to surround an ultrasonic transducer provided on the front end of the insertion portion, and an ultrasonic wave transmission medium is filled in the balloon to transmit an ultrasonic wave.

In a balloon endoscope to which the balloon is attached, a fluid supply/suction port is formed in a position where the balloon is attached. For example, in the endoscope for observing the small intestine or the large intestine, the supply/suction port is formed on the outer circumferential surface of the front end portion (which is referred to as a front-end hard portion or a front-end construction portion) of the insertion portion, and the fluid is supplied and suctioned through the supply/suction port (see JP 2004-329645 A).

However, the balloon endoscope having the supply/suction port cannot be used in a state where the balloon is not attached thereto. That is, when the balloon endoscope is used in the state where the balloon is not attached thereto, a body fluid and the like enter through the supply/suction port and then are attached to the inside thereof. As a result, such a problem arises that it is not possible to accurately control pressure of the balloon due to change in a pressure loss upon using the endoscope with the balloon later on. In order to solve the above-described problem, it may be conceived that the supply/suction port is sealed by a tape and the like. However, in that case, such problems arises that the tape may be peeled off during use, that the body fluid may enter therein because the supply/suction port cannot be completely sealed by the tape and the like, and that a user may have a burden because the tape needs to be prepared additionally.

In view of such a background, the balloon endoscope which can also be used as a general endoscope by closing the supply/suction port has been demanded. Particularly, in the case where the large intestine is observed, there is a case where the general endoscope is used at the first time of an examination and the balloon endoscope is used only at the time when an insertion is difficult. In such a case, when both of the general endoscope and the balloon endoscope are prepared, since it takes time and efforts in a cleaning operation, there is a demand that the balloon endoscope is intended to be used as the general endoscope.

In accordance with the preamble of the patent claim, WO 2006/129419 A1 discloses an endoscope, wherein a check valve member is attached to the insertion portion in order to cover the opening thereof such that fluid can only be supplied into the balloon whereas the fluid is prevented from flowing out of the balloon. As an alternative of this prior art check valve attached to the insertion portion, there is disclosed a check valve formed as a membrane disposed within the hollow insertion portion.

EP 1 857 040 A1 discloses an endoscope having an insertion portion, a balloon attached to the insertion portion, wherein an opening is formed in a surface of the insertion portion to supply a fluid to an inside of the balloon. In order to increase the degree of freedom of a mounting location of the balloon, there are provided two openings in the surface of the insertion portion, each opening being associated with a circumferential groove. One of the end portions of the balloon rests within one of the grooves, thereby closing on of the two openings, while maintaining the other opening in communication with the inside of the balloon.

### SUMMARY OF THE INVENTION

The invention has been made in view of the above circumstances and provides an endoscope which can be used as the general endoscope and the balloon endoscope.
(1) According to an aspect of the invention, an endoscope includes the features of claim 1.
   In accordance with the preamble of the patent claim, WO 2006/129419 A1 discloses an endoscope, wherein a check valve member is attached to the insertion portion in order to cover the opening thereof such that fluid can only be supplied into the balloon whereas the fluid is prevented from flowing out of the balloon. As an alternative of this prior art check valve attached to the insertion portion, there is disclosed a check valve formed as a membrane disposed within the hollow insertion portion.
   With this configuration, since the shutter member is provided in the opening, it is possible to open/close the opening just by moving the shutter member. Accordingly, the endoscope can be used as a balloon endoscope when the opening is opened to the outside, and also can be used as a general endoscope when the opening is closed.
(2) Also, in the endoscope of (1), the shutter member is slidable on the surface of the insertion portion. A hole is formed in the shutter member to pass through the shutter member. When the shutter member is located in the open position, the hole is disposed in a position of the opening and the opening is opened to the outside through the hole. When the shutter member is located in the closed position, the hole deviates from the position of the opening and the shutter member closes the opening.
   With this configuration, since the opening can be opened/closed by sliding the shutter member, it is possible to carry out a switching operation between a balloon endoscope and a general endoscope.
(3) Also, in the endoscope of (2), the insertion portion may have an annular concave portion that is formed in an outer circumferential surface thereof to extend in a circumferential direction. The opening is formed in the annular concave portion. The shutter member may be formed in a ring shape and be rotatably attached to the annular concave portion.
   With this configuration, since the shutter member having the ring shape is attached to the annular concave portion of the insertion portion so as to be rotatable, it is possible to open/close the opening by rotating the shutter member. Thus, it is possible to carry out the switching operation between the balloon endoscope and the general endoscope.
(4) Also, in the endoscope of (3), a side surface of the annular concave portion may be formed in a taper shape. A surface of the shutter member that faces the side surface of the annular concave portion may be formed in a taper shape.
   With this configuration, since the side surface of the annular concave portion and the side surface of the shutter member are formed in the taper shape, a gap between the side surfaces is formed in a V shape. Thus, it is possible to prevent such a case where a foreign substance or the like are filled in the gap.
(5) In the endoscope of any of (1) and (2), the shutter member may be attached to the insertion portion so as to be movable in an axial direction of the insertion portion.

With this configuration, since the opening can be opened/closed just by moving the shutter member in the axial direction of the insertion portion, it is possible to carry out the switching operation between the balloon endoscope and the general endoscope.

According to any of the above configurations, since the shutter member is disposed in the opening, it is possible to open/close the opening just by moving the shutter member. Accordingly, it is possible to use the endoscope as a balloon endoscope when the opening is opened to the outside and to use the endoscope as a general endoscope when the opening is closed.

### BRIEF DESCRIPTION OF THE DRAWINGS

Fig. 1 is a view illustrating a system configuration of a double balloon type endoscope according to an embodiment of the invention.
Fig. 2 is a perspective view illustrating a front end portion of an insertion portion of the endoscope.
Fig. 3 is a section view illustrating a front end in a position where an opening is formed.
Fig. 4 is a section view taken along the line 4-4 shown in Fig. 3.
Fig. 5 is a section view when a shutter member shown in Fig. 4 is moved.
Fig. 6 is a view illustrating an insertion method of a double balloon type.
Fig. 7 is a perspective view illustrating a front end portion according to a second embodiment.
Fig. 8 is a section view illustrating the front end portion shown in Fig. 7.
Fig. 9 is a section view illustrating a shutter member not covered by the invention as claimed and having a different shape from that shown in Fig. 8.

### DETAILED DESCRIPTION OF EMBODIMENTS OF THE INVENTION

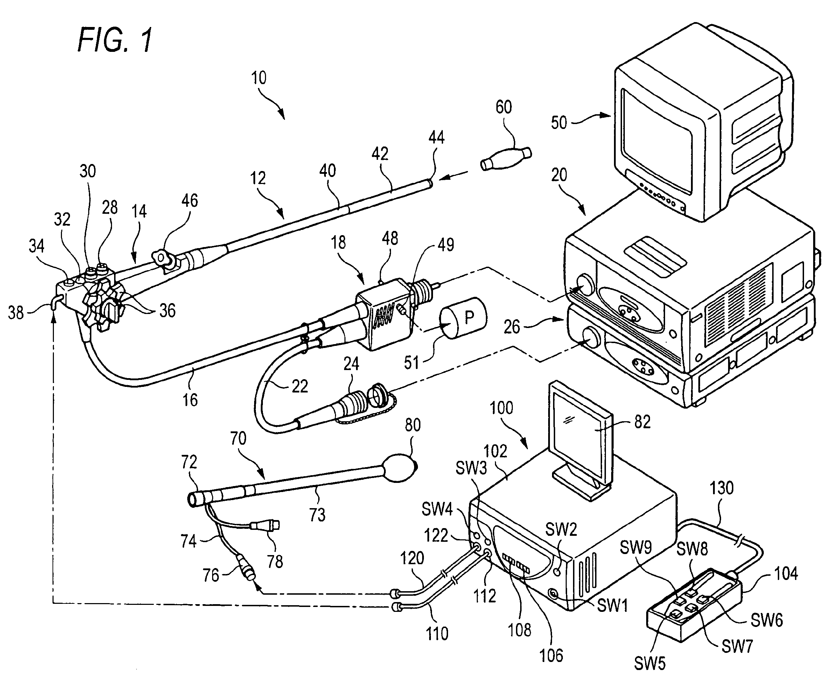

Hereinafter, an endoscope and a method for using the endoscope according to an exemplary embodiment of the invention will be described in detail with reference to the accompanying drawings. Fig. 1 is a view illustrating a system configuration of a double balloon type endoscope in the case where an endoscope 10 according to the embodiment of the invention is used as a balloon endoscope. Additionally, in the case where the endoscope 10 according to the embodiment of the invention is used as a general endoscope, a first balloon 60, an insertion assisting tool 70, a monitor 82 exclusively for the balloon, a balloon control device 100, and the like shown in Fig. 1 are not necessary. The insertion assisting tool 70, which will be described later, may be used to maintain an S-shaped colon in a linear shape.

As shown in Fig. 1, the double balloon type endoscope mainly includes the endoscope 10, the insertion assisting tool 70, and the balloon control device 100.

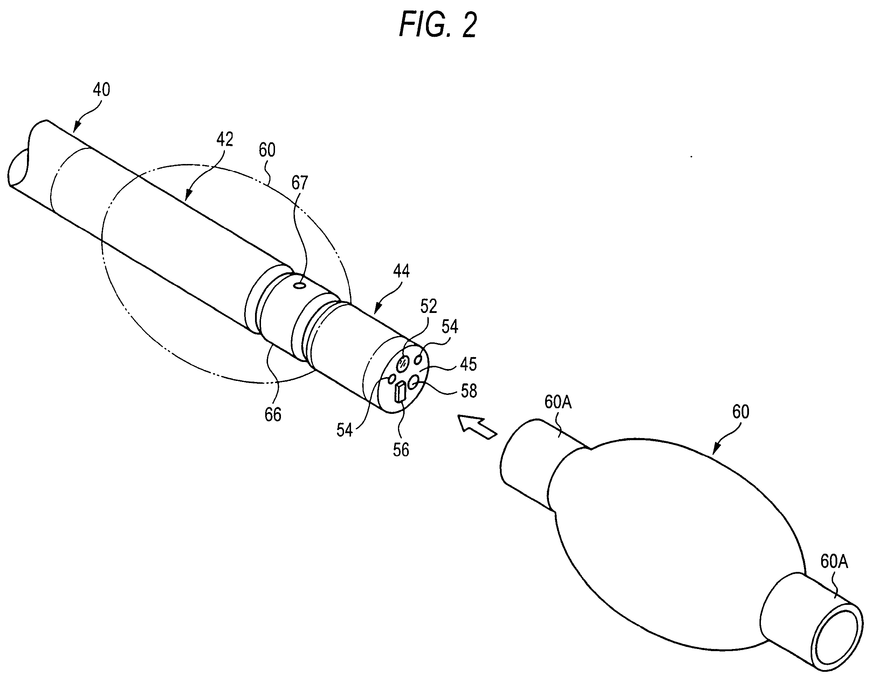

The endoscope 10 includes a hand-side operation unit 14 and an insertion portion 12 that is connected to the hand-side operation unit 14 and is inserted into a body. The hand-side operation unit 14 is connected to a universal cable 16, and an LG connector 18 is disposed at the front end of the universal cable 16. The LG connector 18 is detachably connected to a light source device 20, so that illumination light is transmitted to illumination optical systems 54 (see Fig. 2). Also, an electric connector 24 is connected to the LG connector 18 through a cable 22, and the electric connector 24 is detachably connected to a processor 26.

In the hand-side operation unit 14, an air/water supply button 28, a suction button 30, a shutter button 32 and a function switch button 34 that are disposed together, and a pair of angle knobs 36, 36 are provided. A balloon air supply port 38 that is formed of a tube bent in an L shape is formed on the base end of the hand-side operation unit 14. The first balloon 60, which will be described later, can be inflated or deflated by supplying a fluid such as air to the balloon air supply port 38 or suctioning the fluid from the balloon air supply port 38.

The insertion portion 12 includes a flexible portion 40, a bending portion 42, and a front end portion 44 that are disposed in this order from a side of the hand-side operation unit 14. The bending portion 42 is remotely operated to bend by rotating the angle knobs 36, 36 disposed on the hand-side operation unit 14. Accordingly, the front end portion 44 can be directed in a desired direction.

As shown in Fig. 2, an observation optical system 52, the illumination optical systems 54, 54, an air/water supply nozzle 56, and forceps port 58 are formed in a front end surface 45 of the front end portion 44. A CCD (not shown) is disposed on the rear side of the observation optical system 52 and a signal cable (not shown) is connected to a board for supporting the CCD. The signal cable is inserted through the insertion portion 12, the hand-side operation unit 14, and the universal cable 16, and the like shown in Fig. 1 to extend to the electric connector 24 and is connected to the processor 26. Consequently, an image obtained by the observation optical system 52 is formed on a light receiving surface of the CCD and converted into an electrical signal, and the electrical signal is output to the processor 26 through the signal cable and converted into an image signal. In this way, the observed image is displayed on a monitor 50 connected to the processor 26.

A light-emitting end of a light guide (not shown) is disposed on the rear side of the illumination optical systems 54, 54 shown in Fig. 2. The light guide is inserted through the insertion portion 12, the hand-side operation unit 14, and the universal cable 16 shown in Fig. 1 so that the light incident end is disposed in the LG connector 18. Accordingly, the LG connector 18 is connected to the light source device 20, so that illumination light from the light source device 20 is transmitted to the illumination optical systems 54, 54 through the light guide and is radiated from the illumination optical systems 54 to the front side.

The air/water supply nozzle 56 shown in Fig. 2 communicates with a valve (not shown) operated by the air/water supply button 28 shown in Fig. 1. Also, the valve communicates with the air/water supply connector 48 disposed on the LG connector 18. The air/water supply connector 48 is connected to an air/water supply device (not shown) so that air/water is supplied thereto. Accordingly, air/water can be sprayed from the air/water supply nozzle 56 to the observation optical system 52 by operating the air/water supply button 28.

The forceps port 58 shown in Fig. 2 communicates with a forceps insertion portion 46 shown in Fig. 1. Accordingly, a treatment tool such as a forceps is inserted from the forceps insertion portion 46 so as to be taken out from the forceps port 58. The forceps port 58 communicates with the valve (not shown) operated by the suction button 30. Also, the valve is connected to a suction connector 49 of the LG connector 18. Accordingly, a suction pump 51 is connected to the suction connector 49, and the valve is operated by the suction button 30. Thereby, a lesion part or the like can be suctioned through the forceps port 58.

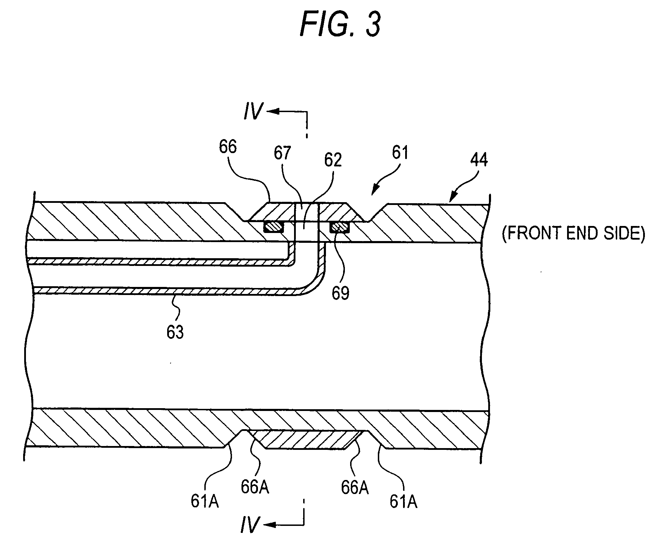

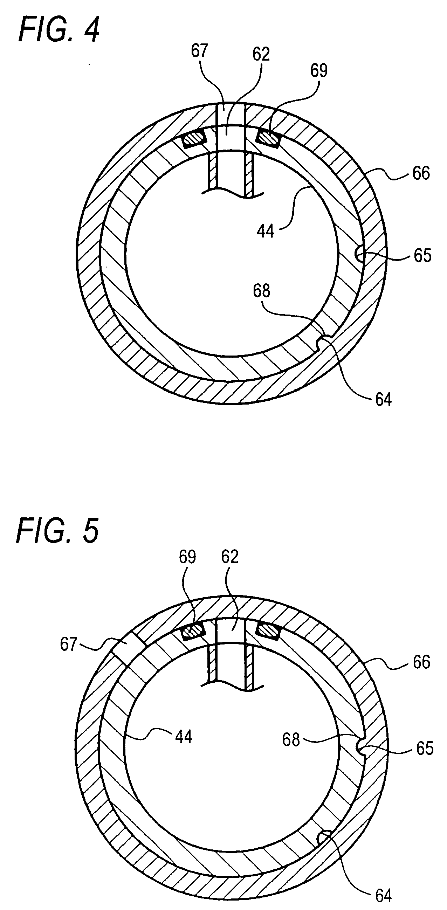

Fig. 3 is a section view of a part of a front end portion 44. Fig. 4 is a section view taken along the line 4-4 shown in Fig. 3. Figs. 3 and 4 show a state where an opening 62 is opened to the outside. Fig. 5 shows a state where a shutter member 66 shown in Fig. 4 is rotated to close the opening 62.

As shown in Fig. 3, the front end portion 44 includes an annular concave groove 61 in the outer circumferential surface thereof. The annular concave groove 61 is formed to extend in the circumferential direction around the front end portion 44. Side surfaces 61A of the annular concave groove 61 are formed in a taper shape. Also, the opening 62 for supplying/suctioning a fluid is formed in the bottom surface of the annular concave groove 61. The opening 62 communicates with a tube 63, the tube 63 communicates with the balloon air supply port 38 shown in Fig. 1, and the balloon air supply port 38 is connected to the balloon control device 100 through a tube 110 which will be described later. Accordingly, it is possible to supply air to the opening 62 and to suction air from the opening 62 by operating the balloon control device 100.

In the annular concave groove 61, a shutter member 66 is disposed. The shutter member 66 is formed in a ring shape. The shutter member 66 is attached thereto with being inserted into the insertion portion 12. The shutter member 66 is configured to slide on the bottom surface of the annular concave groove 61, and to rotate in the circumferential direction about the shaft of the insertion portion 12. The thickness of the shutter member 66 is substantially the same as the depth of the annular concave groove 61. The shutter member 66 is configured so as not to protrude from the outer circumferential surface of the front end portion 44. Furthermore, both side surfaces 66A (surfaces facing the side surface of the annular concave groove 61) are formed in a taper shape.

In the shutter member 66, a through-hole 67 that passes the inner circumferential surface thereof and the outer circumferential surface thereof is formed. The through-hole 67 can be disposed in a position of the opening 62 by rotating the shutter member 66.

As shown in Figs. 4 and 5, a convex portion 68 having a semi-spherical shape is formed in the inner circumferential surface of the shutter member 66. Also, in the bottom surface (outer circumferential surface) of the opening 62 of the front end portion 44, concave portions 64 and 65 which engage with the convex portion 68 are formed. Accordingly, when the shutter member 66 is operated to rotate, as shown in Fig. 4, the convex portion 68 can be disposed in a position where the convex portion 68 engages with the convex portion 64 (hereinafter, referred to as an "open position"). Alternatively, as shown in Fig. 5, the convex portion 68 can be disposed in a position where the convex portion 68 engages with the concave portion 65 (hereinafter, referred to as a "closed position").

In the closed position shown in Fig. 5, a position of the through-hole 67 shifts from that of the opening 62, and thus the opening 62 is closed by the shutter member 66. Accordingly, it is possible to prevent a body fluid and the like from flowing through the opening 62. Thus, it is possible to use the endoscope 10 as a general endoscope. Also, reference Numeral 69 denotes an O-ring which surrounds the vicinity of the opening 62. The O-ring 69 seals a gap between the shutter member 66 and the front end portion 44.

Meanwhile, in the open position shown in Fig. 4, the through-hole 67 of the shutter member 66 is disposed in the position of the opening 62, and the opening 62 communicates with the outside through the through-hole 67. When the opening 62 is opened in this way, it is possible to supply a fluid to the first balloon 60 which will be described later. Thus, it is possible to use the endoscope 10 as a balloon endoscope.

As shown in Fig. 2, the first balloon 60 is made of a thin silicon rubber, etc., and both end portions 60A, 60A are formed in a narrow cylindrical shape. The insertion portion 12 is inserted into the first balloon 60 and the first balloon 60 is disposed in a predetermined position (that is, a position where the through-hole 67 of the shutter member 66 enters the inside of the first balloon 60. Then, the first balloon 60 is attached to the insertion portion 12 by fitting rubber rings (not shown) to the both end portions 60A, 60A. Accordingly, it is possible to inflate/deflate the first balloon 60 by supplying/suctioning air through the through-hole 67 (opening 62). The first balloon 60 is inflated in a substantially spherical shape when air is supplied thereto and is deflated when air is suctioned therefrom so as to adhere to the surface of the insertion portion 12. Furthermore, the balloon may be fixed thereto by winding a thread or the like thereon, instead of using the rubber rings.

Next, in the case where the endoscope 10 according to the embodiment of the invention is used as a balloon endoscope, the insertion assisting tool 70 and the balloon control device 100 which are used together with the endoscope 10 as a double balloon type endoscope will be described.

The insertion assisting tool 70 shown in Fig. 1 includes: a hard gripper portion 72 that has a cylinder shape and that is formed on the base side thereof; and a main body tube 73 that is attached to the front end of the gripper portion 72. The insertion portion 12 of the endoscope 10 is inserted into the main body tube 73 through the gripper portion 72.

The main body tube 73 has a flexible resin tube made of urethane or the like as a base material. The outer circumferential surface and inner circumferential surface of the base material are coated with a hydrophilic coating material (lubricating coating material). As the hydrophilic coating material, for example, polyvinylpyrrolidone is used.

A second balloon 80 is attached to the vicinity of the front end of the main body tube 73. The second balloon 80 is formed in a substantially cylindrical shape so that both ends thereof are narrowed. The second balloon 80 is fixed by winding a thread (not shown) in a state where the insertion assisting tool 70 passes through the second balloon 80. A tube 74 attached on the outer circumferential surface of the insertion assisting tool 70 communicates with the second balloon 80, and a connector 76 is provided on the base end of the tube 74. A tube 120 is connected to the connector 76, which is connected to the balloon control device 100 through this tube 120. Accordingly, the second balloon 80 can be inflated/deflated by supplying/suctioning air with the balloon control device 100. The second balloon 80 is inflated in a substantially spherical shape by supplying air to the second balloon 80, and is attached to the outer circumferential surface of the insertion assisting tool 70 by suctioning air therefrom.

An inlet port 78 is formed on the base end of the insertion assisting tool 70. The inlet port 78 communicates with an opening (not shown) formed in the inner circumferential surface of the insertion assisting tool 70. Accordingly, a lubricant can be supplied to the inside of the insertion assisting tool 70 by injecting the lubricant (for example, water) from the inlet port 78 by an injector or the like. Thereby, when the insertion portion 12 is inserted into the insertion assisting tool 70, it is possible to reduce friction between the inner circumferential surface of the insertion assisting tool 70 and the outer circumferential surface of the insertion portion 12. Accordingly, the insertion portion 12 and the insertion assisting tool 70 can be relatively moved smoothly.

The balloon control device 100 shown in Fig. 1 is a device which not only supplies a fluid such as air to the first balloon 60 and/or suctions the fluid from the first balloon 60, but also supplies a fluid such as air to the second balloon 80 and/or suctions the fluid from the second balloon 80. The balloon control device 100 mainly includes a device main body 102 and a hand switch 104 for remote control.

A power source switch SW1, a stop switch SW2, a first pressure display portion 106, a second pressure display portion 108, a first function stop switch SW3, and a second function stop switch SW4 are provided on the front surface of the device main body 102. The first pressure display portion 106 and the second pressure display portion 108 are panels for displaying a pressure value of the first balloon 60 and a pressure value of the second balloon 80, respectively. If a problem such as a case where the balloon is torn occurs, an error code is displayed on the pressure display portions 106 and 108.

The first function stop switch SW3 and the second function stop switch SW4 are switches for turning ON/OFF a function for endoscope control system and a function for insertion-assisting-tool control system, respectively. When one of the first balloon 60 and the second balloon 80 is not used, the function is turned OFF by operating one of the function stop switch SW3 and the function stop switch SW4 that corresponds to the not-used balloon. In the control system in which the function is turned OFF, the supply and suction of air completely stops. Thus, the corresponding one of the pressure display portion 106 and the pressure display portion 108 is also turned OFF. When both the function stop switches SW3 and SW4 are tuned OFF, an initial setting process or the like can be carried out. For example, calibration with respect to atmosphere pressure is carried out by turning OFF both the function stop switches SW3 and SW4 and simultaneously pressing all the switches SW5 to SW9 of the hand switch 104.

A tube 110 for supplying air to the first balloon 60 and/or suctioning air from the first balloon 60 and a tube 120 for supplying air to the second balloon 80 and/or suctioning air from the second balloon 80 are connected to the front surface of the device main body 102. Backflow prevention units 112 and 122 are provided in connection portions between the tubes 110 and 120 and the device main body 102, respectively, in order to prevent the backflow of the body liquid when the first balloon 60 or the second balloon 80 is torn. The backflow prevention units 112 and 122 are configured in such a manner that a gas-liquid separating filter is inserted into a hollow disk-shaped case (not shown) detachably attached to the device main body 102. In this way, the filters can prevent the body fluid from flowing into the device main body 102.

Also, the stop switch SW5, which is the same as the stop switch SW2 of the device main body 102, the ON/OFF switch SW6 for instructing pressurization/depressurization of the first balloon 60, the pose switch SW7 for maintaining the pressure of the first balloon 60, the ON/OFF switch SW8 for instructing pressurization/depressurization of the second balloon 80, and the pose switch SW9 for maintaining the pressure of the second balloon 80 are formed in the hand switch 104. In addition, the hand switch 104 is electrically connected to the device main body 102 through a cable 130. In the hand switch 104, there is provided a display portion (not shown in Fig. 1) that displays a state of an air supply and/or an air discharge of the first balloon 60 and/or the second balloon 80.

The thus-configured balloon control device 100 supplies air to expand each of the first balloon 60 and the second balloon 80 and also controls the air pressure at a constant value to maintain the inflated state of each of the first balloon 60 and the second balloon 80. In addition, the balloon control device 100 suctions the air from each of the first balloon 60 and the second balloon 80 to deflate each of the first balloon 60 and the second balloon 80 and also controls the air pressure at a constant value to maintain the deflated state of each of the first balloon 60 and the second balloon 80.

The balloon control device 100 is connected to a balloon-dedicated monitor 82. Also, when the balloon control device 100 inflates or deflates each of the first balloon 60 and the second balloon 80, the balloon control device 100 displays the pressure value and the inflated/deflated state of each of the first balloon 60 and the second balloon 80, on the balloon-dedicated monitor 82. Moreover, the pressure value and the inflated/deflated state of each of the first balloon 60 and the second balloon 80 may be displayed on a monitor 50 with being superimposed on the image observed by the endoscope 10.

Next, a method of observing the large intestine using the above endoscope 10 will be described. When the large intestine is observed, the endoscope 10 is first used as a general endoscope. That is, as shown in Fig. 5, the shutter member 66 is moved to the closed position. Also, in the case where the endoscope 10 is used as the general endoscope, since the balloon control device 100 is not used, it is not necessary to connect the tube 110 to the balloon air supply port 38 shown in Fig. 1 and a cap (not shown) may be attached to the balloon air supply port 38.

In the case where the endoscope 10 is used as the general endoscope, the insertion portion 12 is first inserted from an anus, and then the insertion portion 12 is pushed thereinto so that the insertion portion 12 is inserted into the deeper portion of the large intestine. At this time, the S-shaped colon may be maintained in a linear shape by using the insertion assisting tool 70.

In most people, when the insertion portion 12 is pushed thereinto while performing an angle operation, the insertion portion 12 can be inserted into the deeper portion of the large intestine. However, when it is difficult for the general endoscope to be inserted therein due to adhesion of the large intestine, a general insertion method is stopped and a double balloon type insertion method is carried out. That is, the insertion portion 12 is first taken out from the body, and then the general endoscope is switched to the balloon endoscope. The switch operation of the endoscope 10 may be carried out just by rotating the shutter member 66 to move from the closed position to the open position. When the shutter member 66 is disposed in the open position, the opening 62 is opened to the outside through the through-hole 67, the endoscope 10 is switched to the balloon endoscope.

After the endoscope 10 is switched to the balloon endoscope, the first balloon 60 is attached to the insertion portion 12, and the tube 110 is connected to the balloon air supply port 38 of the hand-side operation unit 14. Meanwhile, the tube 120 is connected to the connector 76 of the insertion assisting tool 70 to prepare the insertion assisting tool 70, and the insertion portion 12 is inserted into the insertion assisting tool 70.

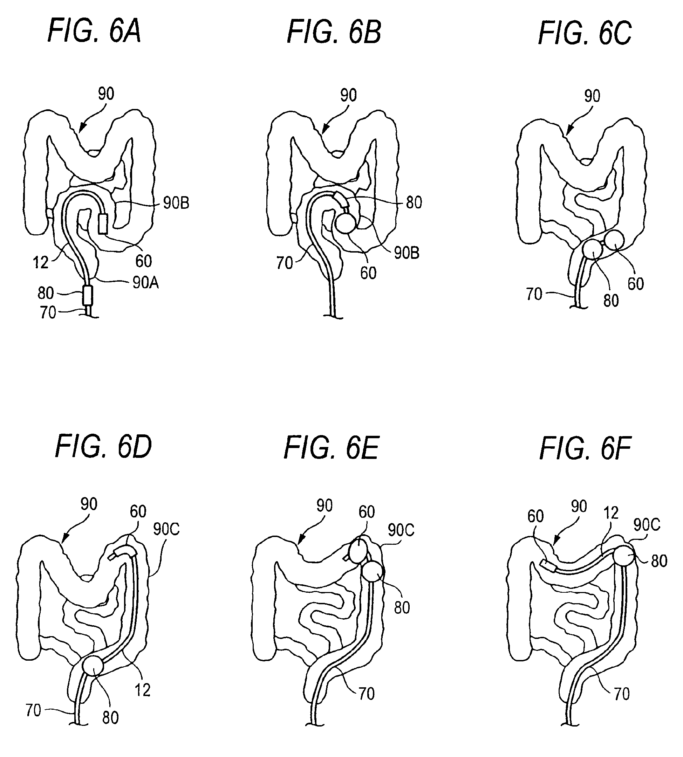

In this state, the double balloon type insertion is started. The double balloon type insertion, as shown in Fig. 6A, is carried out such that the insertion portion 12 is inserted from the anus 90A, and the first balloon 60 is inflated when the front end of the insertion portion 12 reaches the S-shaped colon 90B to allow the front end of the insertion portion 12 to be fixed to the intestine 90 (fixation operation).

Subsequently, as shown in Fig. 6B, the insertion assisting tool 70 is pushed so as to follow the insertion portion 12 (push operation). The front end of the insertion assisting tool 70 is inserted up to the vicinity of the first balloon 60, and then air is supplied to the second balloon 80 so as to be inflated. Accordingly, the second balloon 80 is fixed to the intestine 90, and the intestine 90 is gripped by the insertion assisting tool 70 through the second balloon 80 (grip operation).

Subsequently, as shown in Fig. 6C, the insertion portion 12 and the insertion assisting tool 70 are pulled, so that an extra bending or winding of the intestine 90 is eliminated (pull operation). Then, the air is suctioned from the first balloon 60 to deflate the first balloon 60. In addition, as shown in Fig. 6D, the insertion portion 12 is inserted into the deeper portion of the intestine 90 (for example, up to winding portion of the upper portion of the descending colon 90C) (insertion operation). As described above, the fixation operation of inflating the first balloon 60 and the push operation of the insertion assisting tool 70 following the insertion portion 12 are carried out. Subsequently, the grip operation of inflating the second balloon 80 is carried out, and then the pull operation of the insertion assisting tool 70 is carried out. Accordingly, as shown in Fig. 6E, the extra bending or winding of the intestine 90 is eliminated.

A series of such operations (the insertion operation, the fixation operation, the push operation, the grip operation, and the pull operation) are repeatedly carried out, so that the extra bending or winding of the intestine 90 is eliminated. Accordingly, it is possible to simplify the shape of the intestine 90. Thus, it is possible to insert the front end of the insertion portion 12 into the deeper portion of the intestine 90.

The double balloon type insertion method is carried out such that the insertion portion 12 is inserted in the state where the shape of the intestine 90 is simplified by gripping the intestine 90 with the first balloon 60 and the second balloon 80. Therefore, even if it is difficult to insert the general endoscope, it is possible to securely insert the insertion portion 12.

As described above, since the opening 62 of the endoscope 10 can be opened or closed just by moving the shutter member 66, it is possible to use the endoscope 10 in the form of the general endoscope and the balloon endoscope. Accordingly, according to the embodiment, it is not necessary to prepare two types of endoscopes. Thus, it is possible to facilitate a preparation operation of an examination.

According to this embodiment, since the shutter member 66 is always attached to the insertion portion 12, it is not necessary to prepare a seal portion such as a tape or the like. Thus, it is possible to simply carry out the switching operation between the balloon endoscope and the general endoscope.

According to this embodiment, the side surfaces 66A of the shutter member 66 and the side surfaces 61A of the annular concave groove 61 are formed in the taper shape, and the gap between the shutter member 66 and the annular concave groove 61 is formed in the V shape. Accordingly, a foreign substance cannot be filled therebetween. Thus, it is possible to easily carry out a cleaning operation.

According to this embodiment, since the shutter member 66 is contained inside the annular concave groove 61, the shutter member 66 does not protrude from the outer circumferential surface of the insertion portion 12. Accordingly, a friction between the insertion portion 12 and a body wall decreases. Thus, it is possible to insert the insertion portion 12 smoothly.

In the embodiment described above, the shutter member 66 is moved in the circumferential direction to open or close the opening 62. However, the invention is not limited thereto. For example, the shutter member 66 may be moved in the axial direction to open or close the opening 62. Also, the shape of the shutter member 66 is not limited to the ring shape, but may be any shape which can open or close the opening 62.

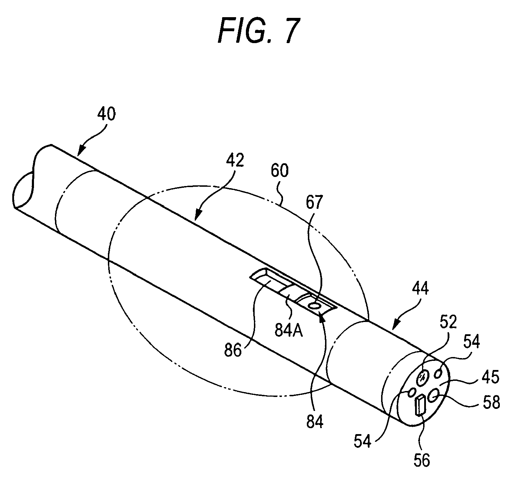

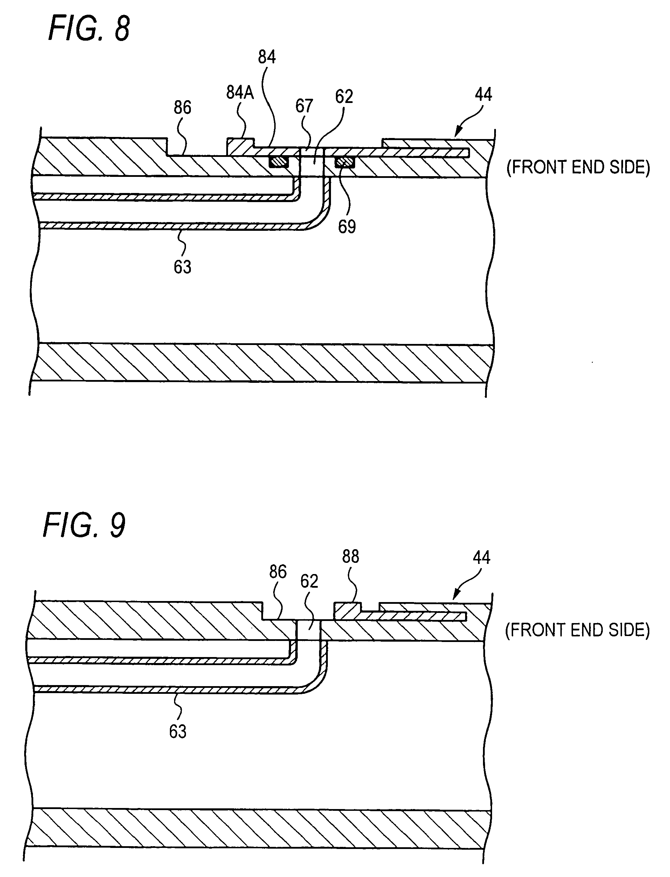

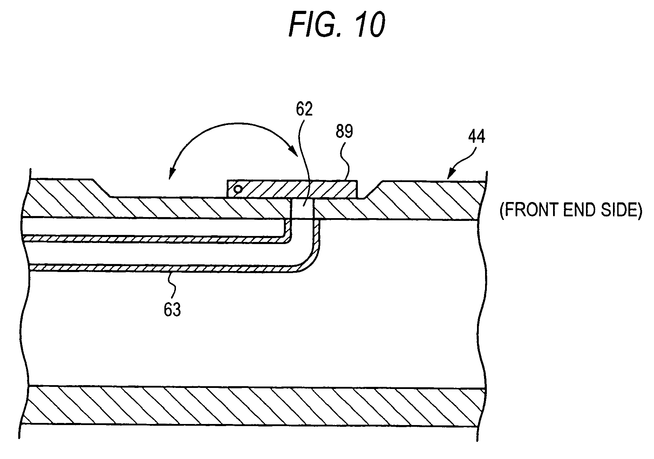

Figs. 7 and 8 are a perspective view and a section view illustrating a shutter member 84 according to a second embodiment.

The shutter member 84 shown in these figures is configured to be slidable in the axial direction of the insertion portion 12. That is, on the surface of the insertion portion 12, a concave portion 86 is formed to extend in the axial direction. The shutter member 84 is received in the concave portion 86, and is attached thereto so as to slide along the concave portion 86 in the axial direction. In the shutter member 84, a through-hole 67 is formed and communicates with the opening 62 in the open position. Also, when the shutter member 84 is moved to the closed position, the position of the through-hole 67 is shifted from that of the opening 62, and the opening 62 is closed by the shutter member 84.

In an end portion of the shutter member 84, a handle portion 84A which protrudes outwardly is formed, and can be gripped when the shutter member 84 is slid. In addition, the shutter member 84 always engages with the insertion portion 12 so as not to be detached therefrom.

Even in the second embodiment with such a configuration, it is possible to open or close the opening 62 just by moving the shutter member 84. Thus, it is possible to carry out the switching operation between the balloon endoscope and the general endoscope.

In the first and second embodiments described above, the opening 62 is opened by positioning the through-hole 67 of the shutter members 66, 84 and the opening 62.

The shutter member 88 shown in Fig. 9 is not covered by the claimed invention.

## Claims

1. An endoscope comprising:
an insertion portion (12);
a balloon (60) attached to the insertion portion (12), wherein an opening (62) is formed in a surface of the insertion portion (12) to supply a fluid to an inside of the balloon (60); and
a shutter member (66, 84) disposed in the opening, wherein
the shutter member (66, 84) is attached to the insertion portion (12) wherein said shutter member is adapted to be movable between (i) a closed position where the shutter member (66, 84, 88, 89) closes the opening and (ii) an open position where the shutter member opens the opening (62) to supply and suck a fluid to and from the inside of the balloon,
**characterised in that**
the shutter member (66, 84) is slidable on the surface of the insertion portion (12),
and
a hole (67) is formed in the shutter member (66, 84) to pass through the shutter member (66, 84),
when the shutter member (66, 84) is located in the open position, the hole (67) is disposed in a position of the opening (62) and the opening (62) is opened to the outside through the hole (67), and
when the shutter member is located in the closed position, the hole deviates from the position of the opening and the shutter member closes the opening (62).

2. The endoscope according to claim 1, wherein
the insertion portion (12) has an annular concave portion (61) that is formed in an outer circumferential surface thereof to extend in a circumferential direction,
the opening (62) is formed in the annular concave portion (61), and
the shutter member (66) is formed in a ring shape and is rotatably attached to the annular concave portion (61).

3. The endoscope according to claim 2, wherein
a side surface of the annular concave portion (61) is formed in a taper shape,and
a surface of the shutter member (66) that faces the side surface of the annular concave portion (61) is formed in a taper shape.

4. The endoscope according to claim 1, wherein the shutter member (84) is attached to the insertion portion (12) so as to be movable in an axial direction of the insertion portion (12).

## Patentansprüche

1. Endoskop, umfassend:
einen Einführabschnitt (12);
einen Ballon (60), der an dem Einführabschnitt (12) befestigt ist, wobei in einer Oberfläche des Einführabschnitts (12) eine Öffnung (62) zum Zuführen eines Fluids in das Innere des Ballons (60) ausgebildet ist; und
ein in der Öffnung befindliches Verschlusselement (66, 84), wobei
das Verschlusselement (66, 84) an dem Einführabschnitt (12) befestigt ist, wobei das Verschlusselement dazu ausgebildet ist, bewegbar zu sein zwischen (i) einer geschlossenen Stellung, in der das Verschlusselement (66, 84, 88, 89) die Öffnung verschließt, und (ii) einer Offenstellung, in der das Verschlusselement die Öffnung (62) freigibt, um ein Fluid in das Innere des Ballons zu leiten oder aus dem Inneren des Ballons abzusaugen,
**dadurch gekennzeichnet, dass**
das Verschlusselement (66, 84) auf der Oberfläche des Einführabschnitts (12) verschiebbar ist, und
in dem Verschlusselement (66, 84) ein Loch (67) ausgebildet ist, welches das Verschlusselement (66, 84) durchsetzt,
wobei, wenn das Verschlusselement (66, 84) sich in der geöffneten Stellung befindet, das Loch (67) sich an einer Stelle der Öffnung (62) befindet und die Öffnung (62) durch das Loch (67) hindurch nach außen geöffnet ist, und
wenn das Verschlusselement sich in der Schließstellung befindet, das Loch von der Lage der Öffnung abweicht und das Verschlusselement die Öffnung (62) verschließt.

2. Endoskop nach Anspruch 1, bei dem
der Einführabschnitt (12) einen ringförmigen konkaven Abschnitt (61) aufweist, der in seiner Außenumfangsfläche ausgebildet ist und sich in Umfangsrichtung erstreckt,
die Öffnung (62) in dem ringförmigen konkaven Abschnitt (61) ausgebildet ist, und
das Verschlusselement (66) in einer Ringform ausgebildet und drehbar an dem ringförmigen konkaven Abschnitt (61) angebracht ist.

3. Endoskop nach Anspruch 2, bei dem
eine Seitenfläche des ringförmigen konkaven Abschnitts (61) in konischer Form ausgebildet ist, und
eine Oberfläche des Verschlusselements (66) gegenüber der Seitenfläche des ringförmigen konkaven Abschnitts (61) konisch ausgebildet ist.

4. Endoskop nach Anspruch 1, bei dem das Verschlusselement (84) an dem Einführabschnitt (12) derart angebracht ist, dass es in axialer Richtung des Einführabschnitts (12) bewegbar ist.

## Revendications

1. Endoscope comprenant :
une partie d'introduction (12) ;
un ballonnet (60) fixé à la partie d'introduction (12), dans lequel une ouverture (62) est formée dans une surface de la partie d'introduction (12) afin de fournir un fluide à un intérieur du ballonnet (60), et
un élément obturateur (66, 84) disposé dans l'ouverture,
dans lequel l'élément obturateur (66, 84) est fixé à la partie d'introduction (12),
dans lequel ledit élément obturateur est apte à être déplacé entre (i) une position fermée, où l'élément obturateur (66, 84, 88, 89) ferme l'ouverture et (ii) une position ouverte, où l'élément obturateur ouvre l'ouverture (62), pour alimenter un fluide vers l'intérieur du ballonnet, et l'aspirer hors de celui-ci,
**caractérisé en ce que**
l'élément obturateur (66, 84) peut être coulissé sur la surface de la partie d'introduction (12), et
un trou (67) est formé dans l'élément obturateur (66, 84) pour passer à travers l'élément obturateur (66, 84),
lorsque l'élément obturateur (66, 84) se trouve dans la position ouverte, le trou (67) est disposé dans une position de l'ouverture (62) et l'ouverture (62) est ouverte vers l'extérieur par le biais du trou (67), et
lorsque l'élément obturateur se trouve dans la position fermée, le trou dévie de la position de l'ouverture et l'élément obturateur ferme l'ouverture (62).

2. Endoscope selon la revendication 1, dans lequel
la partie d'introduction (12) présente une partie concave annulaire (61) formée dans une surface circonférentielle extérieure de celle-ci pour s'étendre dans une direction circonférentielle ;
l'ouverture (62) est formée dans la partie concave annulaire (61), et l'élément obturateur (66) présente une forme en anneau et est fixé de manière à pouvoir tourner sur la partie concave annulaire (61).

3. Endoscope selon la revendication 2, dans lequel
une surface latérale de la partie concave annulaire (61) présente une forme de diminution progressive, et
une surface de l'élément obturateur (66) faisant face à la surface latérale de la partie concave annulaire (61) présente une forme de diminution progressive.

4. Endoscope selon la revendication 1, dans lequel l'élément obturateur (84) est fixé sur la partie d'introduction (12) de manière à pouvoir être déplacé dans une direction axiale de la partie d'introduction (12).
